# EUROPEAN PATENT APPLICATION

(11) **EP 2 840 540 A1**
(43) Date of publication of application: **25.02.2015**
(21) Application number: 13779039.0
(22) Date of filing: 18.04.2013
(51) Int. Cl.: G06Q 10/10, G06Q 50/22

(54) **INTEGRATED MANAGEMENT METHOD AND INTEGRATED MANAGEMENT SYSTEM FOR PUBLIC HEALTH INFORMATION, AND RECORDING MEDIUM THEREFOR**

(30) Priority: 19.04.2012 KR 20120041059
(71) Applicant: Korea Health and Welfare Information Service, Seoul 100-705 (KR); Ministry for Health & Welfare, Sejong 339-012 (KR)
(72) Inventor: HAN, Beum-Soo, Goyang-si Gyeonggi-do 411-730 (KR); MO, Kyoung-Chun, Seoul 131-873 (KR); RYU, Ju-Heon, Hwaseong-si Gyeonggi-do 445-761 (KR); NA, Sang-Il, Seongnam-si Gyeonggi-do 461-803 (KR); CHO, Cheul-Haing, Seoul 132-873 (KR); CHO, Jin-Chang, Gunpo-si Gyeonggi-do 435-723 (KR); LEE, Hyung-Seok, Seoul 134-877 (KR)
(74) Representative: Herrmann, Franz
(86) International application number: PCT/KR2013/003278
(87) International publication number: WO 2013/157861

(57) **Abstract**

Provided are an integrated management method and integrated management system for health information, and a recording medium therefor. According to the present invention, a variety of health information generated during performing health-related projects and administrative services, medical treatment, medical treatment support or the like can be integrated and managed by various health institutes established in each region according to their functions, wherein reference codes generated and managed separately by each health institute are standardized so that integrated business processing and automatic statistical analysis can be carried out, and user-centered atypical analysis can be performed, thereby enabling users of each health institute, a local government or a management agency, to efficiently conduct business transactions and to efficiently create and utilize performance data and statistical data appropriate for each purpose of use.

## Description

### Technical Field

The present invention relates, in general, to an integrated management method and integrated management system for health information, and a recording medium therefor and, more particularly, to an integrated management method and integrated management system for health information, and a recording medium therefor; the method being configured such that a variety of health information generated during the performing of health-related projects and administrative services, medical treatment, medical treatment support or the like can be integrated and managed, according to their functions, by various health institutes established in different regions, wherein reference codes generated and managed separately by each health institute are standardized so that integrated business processing and automatic statistical analysis can be carried out, and user-centered atypical analysis can be performed, thereby enabling users of each health institute or local government and management agency to efficiently conduct business transactions and to efficiently create and utilize performance data and statistical data appropriate for each purpose of use.

### Background Art

Health institutes of various levels, such as health centers, branch offices of the health centers, health medical centers and the like, have been installed in different regions of the country, and thus various kinds of business, such as health-related projects, administrative services, medical treatment, medical treatment support or the like, are being conducted.

In order to manage various kinds of business information, health institutes at the various levels and regions have established and currently operate their own individual information systems, and in the case of these individual information systems, there is a limitation in that they are record-oriented information systems for handling business affairs related specifically to each institute.

For example, since the systems have a limitation in informationizing the services of these health institutes, the overlap of business affairs and the need to perform manual labor frequently occur, and thus by putting pressure on staff in charge of the services, it may take one month or more to calculate and report health-related project results. Thus, it is difficult for the systems to establish and carry out health service plans reflecting the characteristics and needs of differing regions.

In particular, since there is no automatic calculation system for determining result statistics of regional health institute services, the calculation and report of results are performed manually, and it is very difficult for the existing systems to collect and utilize data for policy decisions. Also, in order to calculate statistics needed by users, manual labor is doubly needed.

Furthermore, due to the overlapping development of information systems by each health institute, the lack of information links, the inefficiency of services caused by inefficient management operation, non-standardization of information systems, the lack of compatibility between the information systems, the generation of excessive expenses for system maintenance and management, the vulnerability of personal information and the like, the need to operate and manage a systematic and integrated information system has been raised.

In particular, with respect to a data link with related organizations, since individual systems have been established and operated according to the needs of each health institute, these systems have a limitation in that various data forms and different solutions are applied and the systems are dependent upon the servers of health institutes.

Also, the conventional systems are problematic in that it is impossible to exchange information among health institutes and to calculate statistics because various codes, such as examination codes, conduct codes and the like of health institutes, used in medical treatment and health-related projects are separately managed by the respective health institutes, and in that it is difficult for a person who received medical treatment at one health institute and then visits another health institute to access the details of his or her previous medical treatment.

### Disclosure

### Technical Problem

Accordingly, the present invention has been made keeping in mind the above problems occurring in the prior art, and an object of the present invention is to provide an integrated management method which is configured such that a variety of health information generated during the performing of health-related projects and administrative services, medical treatment, medical treatment support or the like can be integrated and managed, according to their functions, by various health institutes established in different regions, wherein reference codes generated and managed separately by each health institute are standardized so that integrated business processing and automatic statistical analysis can be carried out, and user-centered atypical analysis can be performed, thereby enabling users of each health institute or local government and management agency to efficiently conduct business transactions and to efficiently create and utilize performance data and statistical data appropriate for each purpose of use.

Another object of the present invention is to provide an integrated management system for health information, which is intended to carry out an integrated management method for health information, and a computer readable recording medium intended for implementing the system using a computer.

### Technical Solution

In order to accomplish the above object(s), according to one aspect of the present invention, there is provided an integrated management method for health information which is a method of integrally managing information on at least one service of a service for medical treatment or health-related projects and a service for health administration inputted or requested through a user terminal, and which is performed through an integrated management system for health information connected to at least a user terminal, an operator terminal and a manager terminal via a network, the method comprising: step (a) of registering at least one kind of medical treatment-related reference codes, project-related reference codes, and common codes in a code table through execution of a code management unit, wherein the registering of the code is performed by input through the manager terminal itself, or is performed by receiving approval information inputted through the manager terminal with regard to the code requested to be registered by the operator terminal, and the respective codes are registered so as to belong to at least one code group; and step (b) of inputting, processing and saving at least one kind of service information of medical treatment or health-related project service information and health administration service information through the user terminal based on the codes according to execution of a service information processing unit, wherein the codes are codes for an entire region defined so as to be used by all user terminals of the differing health institutes or any one of codes for each institute defined so as to be used by the user terminals for each health institute.

Meanwhile, according to another aspect of the present invention, there is provided a computer readable recording medium in which a program is recorded, and which is intended for implementing respective steps of the integrated management method for health information.

According to a further aspect of the present invention, there is provided an integrated management system for health information, which is connected to at least a user terminal, an operator terminal and a manager terminal via a network, and which is a system configured to integrally manage at least one kind of service information of medical treatment or health-related project service information and health administration service information inputted or requested through the user terminal. The system includes: a service information processing unit configured to handle one kind of service information of the medical treatment or health-related project service information and the health administration service information inputted or requested through the user terminal; an analysis data generating unit for generating result data or statistical data prepared as a typical or atypical table for services by using the service information; a portal unit linked with a general user terminal intended for using a service processing function of the service information processing unit or using data generated from the analysis data generating unit and configured to handle inputted information or provide requested information; and a code management unit configured to handle the registration, modification and deletion of codes for securing the compatibility of information processed by the service information processing unit, the analysis data generating unit and the portal unit.

### Advantageous Effects

According to the present invention, by integrating information systems which have been separately operated in respective health institutes (health centers, branch offices of the health centers, medical centers and the like) established according to each function/region and informationizing medical treatment services and records, user convenience and service efficiency can be maximally increased, smooth connectivity among services can be secured, and efficiency of information management for administrative services and health-related projects of respective health institutes can be provided through consideration of links among health-related projects/administrative services.

In particular, the present invention is advantageous in that automatic statistical analysis can be performed by standardizing reference codes which are currently individually created and managed according to each health institute, thereby increasing the creation and utilization of health policy data.

Also, since the present invention is configured so that atypical analysis focusing on users can be performed, it is advantageous in that the users of respective health institutes, local governments or management institutes can efficiently create and utilize result and statistical data suitable for each purpose of use.

### Description of Drawings

FIG. 1 is an entire block diagram showing an integrated management system for health information according to one embodiment of the present invention;
FIG. 2 is a detailed block diagram showing the integrated management system for health information according to one embodiment of the present invention;
FIG. 3 is a flow chart showing an integrated management method for health information according to one embodiment of the present invention;
FIG. 4 is a flow chart showing a code registration process resulting from a registration request of an operator terminal with regard to the integrated management method for health information according to one embodiment of the present invention;
FIG. 5 is a flow chart showing code group registration with regard to the integrated management method for health information according to one embodiment of the present invention;
FIG. 6 is a flow chart showing individual or integrated code registration with regard to the integrated management method for health information according to one embodiment of the present invention;
FIG. 7 is a flow chart showing the registration of an operator's authority with regard to the integrated management method for health information according to one embodiment of the present invention;
FIG. 8 is a flow chart showing the registration of a manager's authority with regard to the integrated management method for health information according to one embodiment of the present invention
FIG. 9 is a flow chart showing code attribute registration with regard to the integrated management method for health information according to one embodiment of the present invention;
FIG. 10 is a flow chart showing the creation of a typical or atypical table with regard to the integrated management method for health information according to one embodiment of the present invention;
FIG. 11 is a flow chart showing an atypical table sharing process with regard to the integrated management method for health information according to one embodiment of the present invention; and
FIG. 12 is a flow chart showing a grouping process of analysis viewpoints and analysis indicators with regard to the integrated management method for health information according to one embodiment of the present invention.

### Best Mode

The present invention may be embodied in many alternate forms without departing from the technical ideas or main features of the invention. Accordingly, the present invention should not be construed as limited to the embodiments which are only examples in every respect.

Terms such as a first term and a second term may be used for explaining various elements, but the elements should not be limited to these terms. These terms is used only for the purpose for distinguishing an element from other elements. For example, a first element could be termed a second element, and, similarly, a second element could be termed a first element, without departing from the scope of the present invention. As used herein, the term "and/or" includes any and all combinations of one or more of the associated listed items.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

The terminology used herein is for the purpose of describing particular embodiments only and is not intended to be limiting of example embodiments of the invention. As used herein, the singular forms "a", "an" and "the" are intended to include the plural forms as well, unless the context clearly indicates otherwise. It will be further understood that the terms "comprises", "comprising", "includes" or "including", when used herein, specify the presence of stated features, integers, steps, operations, elements, and/or components, but do not preclude the presence or addition of one or more other features, integers, steps, operations, elements, components, or groups thereof.

Unless otherwise defined, all terms including technical and scientific terms used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinbelow, preferred embodiments of the present invention will be described in detail with reference to the accompanying drawings. In the explanation with reference to the accompanying drawings, regardless of reference numerals of the drawings, like numbers refer to like elements through the specification, and repeated explanation thereof is omitted. In the following description, it is to be noted that, when the functions of conventional elements and the detailed description of elements related with the present invention may make the gist of the present invention unclear, a detailed description of those elements will be omitted.

An integrated management system of the present invention is intended to maximize user convenience and work efficiency and ensure a smooth linkage among business affairs through informatization of medical treatment affairs and medical treatment records resulting from integrating health medical treatment/medical treatment support systems which are currently separately operated in existing health institutes (health centers, branch offices of the health center, health medical centers and the like), and to promote the efficiency of information management for administrative services and health-related projects of each health institute by considering a linkage between the health services/administrative duties.

Table 1 shows the exemplary items of service organizations which are integrated and managed through the integrated management system for health information according to an embodiment and schematically exemplifies the service organizations of medical treatment, health services, and health administration.

**[Table 1]**

| | |
|---|---|
| Health administration (Health center) | General health administration |
| | Pharmaceutical management task |
| | Civil affairs |
| Health administration (Health medical center) | Hospital duty administration |
| | Pharmaceutical management task |
| | Civil affairs |
| Medical treatment duty and medical treatment support (common) | Medical treatment |
| | Medical treatment support |
| Health-related projects (common) | Health promotion projects |
| | Health projects for the elderly |
| | Dental health projects |
| | Administration projects of chronic diseases |
| | Infectious disease control projects |
| | Services for rehabilitation of the disabled |
| | Mother and child health services |
| | Health projects of regions for oriental medicine |
| | Mental health project |
| | Specialized projects |
| | Customized home visiting health services |

Also, Table 2 (civil affairs) and Table 3 (general health administration duties) exemplarily show the administrative affair organizations which are integrated and managed through the integrated management system for health information according to the present embodiment.

**[Table 2]**

| Service Name | Detailed Functions (Examples) | Description of Functions (Examples) |
|---|---|---|
| Civil affairs | Public complaint | Direct appealing |
| | reception and management | persons who visit health centers (health medical centers) into departments in charge of public complaints & handling and managing information on received public complaints |
| | Management of payment receipt and closing accounts | Receive all fees including charges for vaccination, charges for inspection, charges for issuance of all certificates or the like and manage closing accounts therefor |
| | Issuance management of all certificates | Issue and provide all certificates offline or online |

**[Table 3]**

| Service Name | Detailed Functions (Examples) | Description of Functions |
|---|---|---|
| General health administration | Local health care plan | Current state of use according to each kind of insurance, a distribution ratio for top 10 injuries and diseases, distribution of the number of days medical treatment is conducted per year |
| | Medical improvement project in rural areas | General current state of health clinics |
| | Health care center management | Personnel status management for each health clinic |
| | Public health doctor management | Linkage and registration of a current state of public health doctors |

Table 4 (medical treatment service) and Table 5 (medical treatment support) exemplify the organizations of the medical treatment duty which are integrated and managed through the integrated management system for health information according to the present embodiment.

**[Table 4]**

| Service Name | Detailed Functions (Examples) | Description of Functions (Examples) |
|---|---|---|
| Environment setup | Individualized environment setup | Environment setup for each user |
| Patient administration | Target patent management | Register patients according to each type of concern and continuously manage the registered patients |
| Prescription administration | Issuance of prescription | Issue prescriptions (e.g. a drug via injection), examination, physical therapy, oriental medical treatment, dental treatment and like |
| Medical record administration (EMR: Electronic Medical Record) | Recording of information of the first medical examination | Prepare the first medical examination records such as main symptoms, etc. for out-patents and in-patents |
| Nursing record administration (EMR) | Clinical observation | Input the numerical values of blood pressure, weight, height, etc. and check graphs |
| Nursing management | Hospital room management | Request transfer and relocation for inpatients and inquire current states |
| Integrated check | Checking of flow sheets | Inquire medical treatment information on patients according to each day of the week and each day of checkups |
| Test management | Checking of examination results | Inquire results for requested examination, radiation treatments, and the like |
| Surgery management | Surgery schedule management | Manage operation schedules for inpatients and handle necessary matters after operation |
| CP (Critical Pathway) management | Application of CP | Apply critical pathways (CP) to specific CP patients |
| Information query | Checking of the current state of hospital rooms | Check the current state of hospital rooms |

**[Table 5]**

| Service Name | Detailed Functions (Examples) | Description of Functions (Examples) |
|---|---|---|
| Drug/injection | Receiving of administration prescription of medicine | Confirm administration prescription of a medicine prescribed through medical treatment, give a drug/injection or issue prescription |
| Physical therapy | Receiving of physical therapy | Receive of physical therapy prescription |
| Clinical pathology examination | Result management | Input examination results after carrying out the examination, and verify and check the inputted results |
| Radiation | Receiving of radiation | Receive radiation prescription requested in medical treatment/health projects and output name cards |
| Inventory management | Warehousing/delivery management of products | Manage warehousing/delivery request/release control duties of products |
| Integrated reservation management | Integrated reservation management | Check reservation information for entire task |

Table 6 (Health promotion service) and Table 7 (mother and child health service) exemplify task organizations for health services which are integrated and managed through the integrated management system for health information. Examples of the health services are health promotion services, dental health projects, national cancer management projects, health projects for the elderly, chronic disease management projects, mother and child health projects, home visiting health projects, rehabilitation projects for the disabled, infectious disease management projects, mental health projects, medical checkup projects for oriental medicine and the like, and examples of administrative services are general health administration services, civil affairs and the like.

**[Table 6]**

| Service Name | Detailed Functions (Examples) | Description of Functions (Examples) |
|---|---|---|
| Medical check-up | Reception | Register personal information required at the time of registering a request for a medical checkup, the kind of medical checkup and items for the medical checkup |
| Nutrition project | Nutrition management | Register and manage information on medical examinations, dietary intake and eating habits for persons targeted for nutrition management and manage counseling thereof |
| Obesity prevention project | Obesity management | Register and manage the details of medical examination by interviews and a basic medical checkup |
| Project for smoking prohibition and moderation in drink | Management for no smoking | Manage consult content required to manage target persons (consultation date, the contents of management, etc.) |
| Exercise project | Exercise management | Manage schedules for persons required to exercise and their result values before and after exercise |

**[Table 7]**

| Service Name | Detailed Functions (Example) | Description of Functions (Example) |
|---|---|---|
| Pregnant women registration and management | Registration of target persons | Register pregnant women, perform health screening, input childbirth information, distribute nutritional supplements, and manage records regarding discharge from hospitals |
| Infertile couple management | Registration of target persons | Support surgical procedure fees for infertile couples and manage information on infertile couples |
| Helper management for postpartum women and newborn babies | Information management of target persons | Manage information on target persons, and output bills for supporting medical expenses when the target persons are postpartum women in mountain and island regions |
| Pregnant women-related output management | Statistics | Manage statistics, current states, reports, notifications (health records, paper forms, etc.) and other individual or entire outputs |
| Infant registration management | Registration management of target persons | Manage personnel information, health examinations, health checkups (six months old, eight months old, three years old and six years old), protective inoculation, distribution of nutritional supplements, record information on a discharge from hospitals which are necessary for infant registration management |
| Test management for congenital metabolic disorders | Registration of target persons | Manage matters regarding tests for congenital metabolic disorders (test requests, test results, supporting of fees for taking blood samples, specially prepared dry milk, medical expenses and the like) |
| Premature and disfigured baby management | Registration of target persons | Manage matters regarding premature and disfigured babies (baby information, baby state, management contents, supporting dates, supporting amounts and the like) |
| Infant growth and development screening | Registration of target persons | Register and input information on developmental assessment necessary for infant growth and development management (personal and social light exercise, development of adaptability, language and motor skills), anthropometric investigation (weight, height, girth of the head, girth of the chest, etc.), checkups (body, oral cavity, eyesight, hearing, etc.) |
| Infant-related output management | Statistics | Manage statistics, current states, reports, notifications (health records, paper forms, etc.), and other individual or entire outputs required according to each infant-related duty (function) |

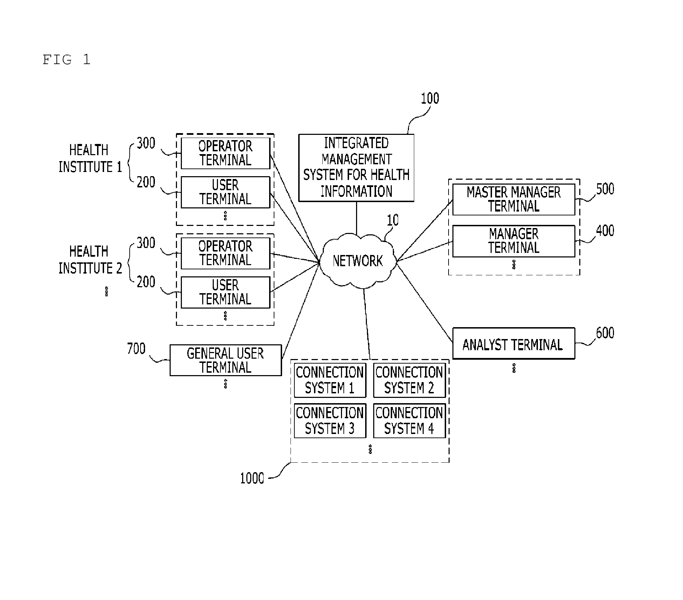

FIG. 1 is an entire block diagram of an integrated management system for health information according to one embodiment of the present invention.

The integrated management system for health information according to the present embodiment is connected to user terminals 200, operator terminals 300, a manager terminal 400 via a network 10.

The 'user terminals 200' mean the terminals of users who work in health institutes at various levels, such as health centers, branch offices of the health centers, health medical centers and the like which are installed per each region across the country and are terminals used to input, check and save so-called operational duty information for medical treatment or health services, health administration and the like. The 'users' may be, for example, administrative staff members of health centers, staff members at hospital administrations of health medical centers, nurses, doctors and the like.

The 'operator terminals 300' may refer to terminals of persons in charge of data processing who work in health institutes of various levels, such as health centers, branch offices of the health centers, health medical centers and the like installed per each region across the country. The operator is entitled to request the integrated management system to register, modify, delete or the like codes related to duties of the corresponding health institutes.

As exemplified in FIG. 1, one operator terminal 300 and multiple user terminals 200 may be provided in each health institute, but the number of the operator terminal is not limited to one.

The 'operator terminal 400' may refer to, for example, a terminal of a manager of the integrated management system for health information, namely, a terminal of a manager who is entitled to handle the registration, modification and deletion of codes with regard to the present embodiment.

The 'network (10)' may refer to a general communication network such as the internet or intranet and may be understood as covering all wired and wireless networks.

A 'master manager terminal 500' illustrated in FIG. 1 may refer to a terminal of a master manager who can register the authority of a manager among manager terminals, namely, a terminal of a master manager who is entitled to carry out comprehensive duties regarding the setting and using of codes of the integrated management system for health information, the setting of authority, and the like including duties such as the registration of codes, the registration of code groups, the registration of organizational trees which can be performed by a general manager.

An 'analyst terminal 600' illustrated in FIG. 1 may be refer to a terminal of an analyst who analyzes and uses result data or statistical data prepared as a typical or atypical table, for example, a terminal of a staff person in charge of analysis who is engaged in public health policy duties and works in a public health related institute or government department.

A 'general user terminal 700' illustrated in FIG. 1 may refer to a terminal of a general user who uses medical treatment and medical project services provided from health institutes of various levels through the integrated management system for health information 100. For example, general users may connect to the integrated management system for health information 100 and use desired services such as reservation, search, and confirmation of the details of use and the like. Table 8 exemplifies the organization of portal services for the public which is integrally managed through the integrated management system for health information according to the present embodiment, namely, portal services for the public carried out through the general user terminal.

**[Table 8]**

| Service Name | Detailed Functions (Examples) | Description of Functions (Examples) |
|---|---|---|
| Sanitation information | Introduction of health centers | Define health centers and guide the task of health centers , the services of health centers, and the civil affairs of health centers |
| Health information | e-healthy country information | Define the content, symptoms and prescription of 100 common diseases |
| e-health center | Issuance of all certificates | Guide issuance of all certificates and issue the certificates |
| Customer participation | Q & A | Provide the service for a free bulletin board regarding all portal services for the public |
| Reference library | Statistical data | Provide the service for a reference library regarding Ministry of Health and welfare statistics |
| News | Health and medical news | Provide the service for healthcare-related medical news |

Meanwhile, the 'terminal' means a terminal having a logical concept which serves as a client in a usual server-client environment, but does not necessarily means a separate physical terminal.

A 'connection system 1000' illustrated in FIG. 1 may mean systems of various relevant institutes which are required to be connected to each other in order to integrally manage health information. Examples of these relevant institutes are the Centers for Disease Control and Prevention, the National Cancer Center, the National Health Insurance Corporation, the Health Insurance Review & Assessment Service, the Korean National Tuberculosis Association, local governments and the like.

The integrated management system for health information 100 according to the present embodiment is connected to the respective terminals or the connection system via the network according to the configurations and integrally manages information on at least one aspect of medical treatment or health-related projects, and health administration inputted or requested through the user terminal 200. Table 9 exemplarily shows the organization of connected services which are integrally managed through the integrated management system according to the present embodiment and exemplifies the details of services which are required to be linked with each connection system.

**[Table 9]**

| Connected Services (Examples) | Connected Institutes | Transmission | Reception | Connection Patterns |
|---|---|---|---|---|
| Connection with information on recipient of national basic livelihood | Safety Executive | Administrative information systems of local governments | Integrated management system for health information | Web Service |
| Connection with vaccination registration information | Centers for Disease Control and Prevention | Integrated management system for health information | System for protective inoculation registration | API |
| Connection with the registration of tuberculosis patients | National Tuberculosis Association | Integrated management system for health information | Tuberculosis surveillance system | Web Service |
| Connection with information on qualification for insurance | National Health Insurance Corporation | Qualification management system for medical aid | Integrated management system for health information | Web Service |
| Connection with early cancer screening test results | National Cancer Center | Information system for national early cancer screening projects | Integrated management system for health information | Web Service |
| Connection of persons targeted for no smoking and the details of project execution | Institute for Health and Social Affairs | System for no smoking clinics | Integrated management system for health information | EAI-HTTP |
| Integrated system for patients with rare and intractable diseases | Centers for Disease Control and Prevention | Integrated system for patients with rare and intractable diseases | Integrated management system for health information | Connection with screen |
| Real name verification of foreigners | KAIT | KAIT system | Integrated management system for health information | API |

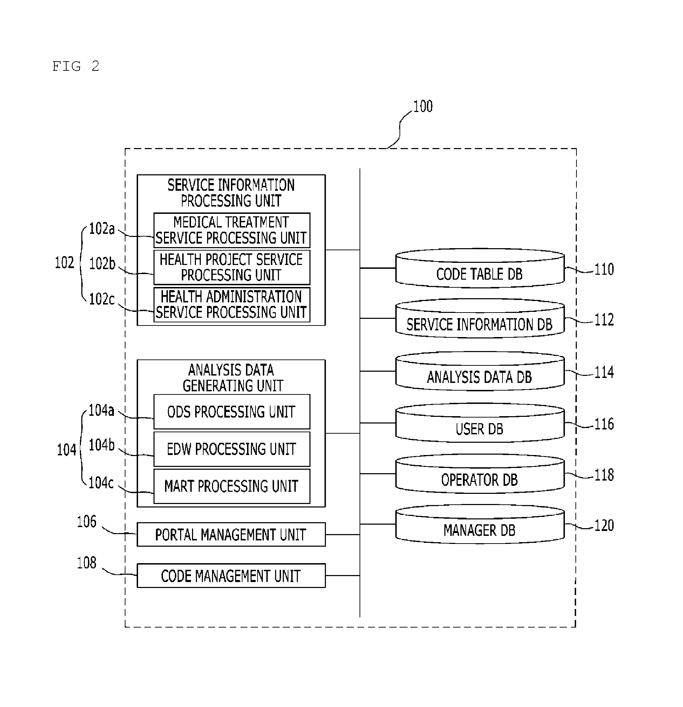

FIG. 2 is a detailed block diagram showing the integrated management system for health information according to one embodiment of the present invention.

The integrated management system for health information 100 according to the present embodiment includes a service information processing unit 102 intended for processing service information on any one of medical treatment or health-related projects and health administration inputted or requested through the user terminal 200. According to its detailed functions, the service information processing unit 102 includes a medical treatment service processing unit 102a, a health-related project service processing unit 102b and a health administration service processing unit 102c.

The integrated management system for health information 100 according to the present embodiment also includes an analysis data generating unit 104 intended for generating result data or statistical data prepared as a typical or atypical table. According to its detailed functions, the analysis data generating unit 104 includes an ODS (Operational Data Store) processing unit 104a, an EDW (Enterprise Data Warehouse) processing unit 104b, a mart processing unit 104c. The detailed processing processes of each unit will be described later.

Table 10 and Table 11 exemplarily show the organizations of a result report and a statistical report created as typical tables in the integrated management system for health information according to the present embodiment.

**[Table 10]**

| Division | Service Name (Example) | Result Name (Example) |
|---|---|---|
| Result Report for Health Administration | Health and medical treatment plan for administrative areas | Current state of insurance use according to each kind of insurances |
| | | Distribution of the number of days of medical treatment per year for hypertensive/diabetic patients |
| | Current state of medical services | Sickbed management and medical treatment pattern |
| Result Report for | Dental health | Result reports of dental |
| Health Projects | | health rooms |
| | | Results of elderly oral health |
| | Infectious disease management project | Current state of tuberculosis examination reception and results |

**[Table 11]**

| Division | Service Name (Examples) | Name of Results (Examples) |
|---|---|---|
| Health-related Proj ect | Health Promotion Project | Statistics of medical checkups (according to each disease and each region) |
| | Infectious disease management project | Current state of protective inoculation |
| | Chronic disease management project | Current state of diseases according to each age group |
| | Dental health project | Current state of checkups and investigations of health conditions according to each target person |
| Medical Treatment | Medical Treatment | Current state of outpatients according to each insurance |
| | | Statistics for frequency of each prescription |

Table 12 exemplarily shows the organization of an actual report or a statistical report which can be created as an atypical table in the integrated management system for health information according to the present embodiment.

**[Table 12]**

| Service Name (Example) | Offering State (Example) |
|---|---|
| Customized home visiting health management project | Current state of health levels of vulnerable social groups |
| | Current state of elderly health care |
| Mother and child health project | Current state of the registration at health centers of pregnant women and infants |
| | Current state of premature congenitally abnormal babies |
| Mental health project | Current state of local mental health projects |
| | Current state of counseling with children/teens/adults |

The integrated management system for health information 100 according to the present embodiment also includes a portal management unit 106 liked with the general user terminal 700 of general users who want to use a service processing function or to use generated data of analysis data generating unit 102 and configured to handle inputted information or to provide requested information.

The integrated management system for health information 100 according to the present embodiment also includes a code management unit 108 configured to handle the registration, modification and deletion of codes for securing the compatibility of information processed in the service information processing unit 102, the analysis data generating unit 104 and the portal management unit 106 and to carry out registration processing for setting the authority of an operator and a manager.

The code management unit 108 functions to at least register one kind of medical treatment-related reference codes, project-related reference codes and common codes in a code table 310.

The medical treatment-related reference codes are reference codes necessary for medical treatment, and the details thereof will be described later. The project-related reference codes mean reference codes for each health-related project such as a protective inoculation code, a chronic disease code and the like. The common codes may mean base codes necessary for processing operational service information such as medical treatment or health-related project services and health administration services and may be, for example, an institute code, a gender code and the like.

The medical treatment-related reference codes have a hierarchical structure composed of single codes defining an item corresponding to one remuneration processing unit, groups defined by a combination of the single codes, and a profile defined by a combination of at least two groups or single codes. Also, the profile may be used at the time of inputting, processing and saving information on the health projects.

The 'single codes' mean, for example, an insurance code (an electronic data interchange (EDI code) designated as a subject for insurance benefits, and the examples thereof are a drug injection code (Western drugs, injections, Chinese drugs or the like), an examination code (clinical pathology examination, radiographic inspection or the like), a treatment code (medical treatment, dental treatment, oriental medical treatment, physical therapy or the like), a fee code(fees for all certificates and fees for each care visit) and other codes.

The 'groups' are defined by a combination of the single codes for users' convenience, and the examples thereof are a clinical pathology examination group, a radiographic inspection group and the like.

The 'profile' is composed of a combination of the groups (examination groups) or a combination of respective items of the single codes and is used for service information processing in the field of health-related projects because it has a characteristic appropriate for being matched with project services in the field of, in particular, the health-related projects. The profile is composed of, for example, N groups and M sign code items and is provided for user convenience. The profile may be understood in greater detail as exemplified in Table 13.

**[Table 13]**

| Profile | Group | Single Code |
|---|---|---|
| Medical examination Report | Tests for AIDS | HIV antibodies and others |
| | | HIV antibodies, HIV antigen/antibody screening inspection |
| | Tests for hepatitis B | Hepatitis B surface antigen and others |
| | | Hepatitis B surface antibody and others |
| | Liver function tests of two kinds | AST[SGOT] |
| | | ALT[SGLT] |
| | Chest X-ray | Chest [direct] 1 sheet |
| | | 14*17 film |

Also, the single codes may be defined as including a first single code defining a subject item for insurance benefits corresponding to the insurance code and a second single code defining items that is added as a subject for the single code by going through approval and input of the manager terminal 400 and is not based on the insurance code.

Each of Tables 14 to 18 shows one example of a code configuration (an examination code, a treatment code, a medicine code, a common code, a code for each institute) of the integrated management system for health information according to the present embodiment of the invention. Tables 14 to 17 may be deemed as showing examples of the first single code, and Table 18 may be deemed as showing examples of the second single code.

**[Table 14]**

| Name of Institutes | Before establishing the integrated management system for health information | | After establishing the integrated management system for health information | |
|---|---|---|---|---|
| | Previous Code | Previous Code Name | New Code | New Code Name |
| AA-county Health Center and County hospital | MI00350 | CLO Test | WB4151 | Helicoba cterpylori Test(dur ing endoscopy)- CLO Test(ure asetest) |
| BB-county Health Center and Country Hospital | OT00180 | Helicobacterpylori test | | |
| CC-county Health Center and Country Hospital | OT00200 | CLO Test during endoscopy | | |
| DD-county Health Center and Country Hospital | SE00590 | Helicobacterpylori endoscopy | | |

**[Table 15]**

| Name of Institutes | Before establishing the integrated management system for health information | | After establishing the integrated management system for health information | |
|---|---|---|---|---|
| | Previous Code | Previous Code Name | New Code | New Code Name |
| AA-county Health Center and Country Hospital | DA02330 | Fit and fissure sealant [per one tooth] | WU2390 | Fit and fissure sealant [per one tooth] |
| BB-county Health Center and | DA01229 | Fit and fissure sealant | | |
| Country Hospital | | [per one tooth] | | |
| CC-county Health Center and Country Hospital | DA02349 | Fit and fissure sealant [per one tooth] (less than full eight years old) | | |

**[Table 16]**

| Name of Institut es | Before establishing the integrated management system for health information | | After establishing the integrated management system for health information | |
|---|---|---|---|---|
| | Previous Code | Previous Code Name | New Code | New Code Name |
| AA-county Health Center and Country Hospital | 424 | Mosapride citrate 5 mg | W6433008 60 | Mosapride citrate 5 mg of Chong Kun Dang Pharmaceutical Corp. - 1 tablet |
| BB-county Health Center and Country Hospital | AH90380 | Mosapride citrate 5 mg | | |
| CC-county Health Center and Country Hospital | BMOC498 | Mosapride citrate 5 mg | | |

**[Table 17]**

| Common Classification Codes (Examples) | Name of Common Classification Codes | Common Codes | Name of Common Codes |
|---|---|---|---|
| 1 | Sex | 0 | Non-description |
| 1 | Sex | 1 | Male |
| 1 | Sex | 2 | Female |
| 1 | Sex | 3 | Other |
| 16 | Reason for cancellation of operation | 1 | Patent's request for postponement |
| 16 | Reason for cancellation of operation | 2 | Patent's refusal of operation |
| 16 | Reason for cancellation of operation | 3 | Need to perform an additional test before operation |
| 16 | Reason for cancellation of operation | 4 | Other |

**[Table 18]**

| Code (Example) | Name | The number of using institutes |
|---|---|---|
| WMD0302671 | Hemocontin Continus Tablets (Korea Pharma)-one tablet | 246 |
| WMD0900029 | Total coliform group | 1524 |
| WMD1000006 | ultrasonography (abdomen) | 25 |

One example of the generative rule of a first single code and a second single code may be understood through Table 19 below.

**[Table 19]**

| Division | Generative Rule | Example |
|---|---|---|
| First single code | - Western drug: W\| \|insurance code-oriental drug: O\| \|insurance code | - W644702910 Renexin tablet (SK chemicals Co., Ltd.- one tablet - WC4610 treponema pallidum hemagglutination assay TPHA |
| Second single code | - Western drug: W\| \|large classification of insurance\| \|number (10 digits)- Oriental drug: O\| \|large classification of insurance\| \|number (10 digits) (large classification of insurance: medicine MD, dental DT, oriental medicine OM, health center HC, health administration HA) | - WMD0900367 specific gravity (urine) - WMD0326036 hemoforce (Joongwea Pharmaceutical Co., Ltd.) - OOM0300488 ostericum koreanum |

In another aspect, the medical treatment-related previous reference codes are composed, according to each health institute, of an attribute portion for all regions whose management authority is possessed by the manager terminal 400, and an attribute portion for each institute whose management authority is possessed by the operator terminals 300.

Each of Tables 20 and 21 show one example for a configuration (a fee master table and an examination attribute table in order) of the medical treatment-related reference codes with regard to the integrated management system for health information according to the present embodiment of the invention.

**[Table 20]**

| Codes of Health institutes | Name of Health institutes | Classification | Medical Treatment Based code (Examples ) | Name of reference codes | Fees (Won) |
|---|---|---|---|---|---|
| 00000 | Master | Drug injection | WA438506 | Tylenol-ER extended release tablet (Janssen Korea Ltd.)- one tablet | 651 |
| | | Examination | WB1040 | The number of red blood cells | 740 |
| | | Measurement | WU0074 | One visit to endodontics [per one root canal]-adult tooth | 24,400 |
| A1201 | 000 Health Center | Drug by injection | WA438506 11 | Tylenol-ER extended release tablet (Janssen Korea Ltd.)- one tablet | 57 |
| | | Examination | WB1040 | The number of red blood cells (RBC) | 740 |
| B1501 | 000 Health Center | Drug by injection | WA438506 11 | Tylenol-ER extended release tablet (Janssen Korea Ltd.)- one tablet | 50 |
| | | Measurement | WU0074 | One visit to endodontics [per one root canal]-adult tooth | 24,400 |
| D1401 | 000 Health Medical Center | Measurement | WU0074 | One visit to endodontics [per one root canal]-adult tooth | 24,400 |

**[Table 21]**

| Codes of Health institutes | Name of Health institutes | Medical treatment related codes (Examples) | Name of Reference codes (Example) | Division of Tests on Consignment |
|---|---|---|---|---|
| 00000 | Master | WB2010 | A,B,O typing - serum | Test performed in hospital |
| A1201 | 000 health center | WB2010 | A,B,O typing - serum | Test on consignment |
| B1501 | 000 health center | WB2010 | A, B, O typing - serum | Test performed in hospital |

It is more efficient that the code attributes (fees and division of a test on consignment) arranged at the column on the most right side of Table 20 or Table 21 are defined according to the characteristics of each health institute at the time of performing the operational services. For example, in the case of some health institutes, in spite of the same drugs prepared by the assistance of corresponding local governments, there may be a difference in the amounts of money charged to patients, and a difference in units for the amount of prescription drugs rendered in consideration of local environments. In consideration of this fact, while the code attributes are basically managed using the attribute portion for all regions, it is preferable that some attributes such as fees, the division of tests on consignment and tests be determined at the level of the hospitals, and the like to be managed using the attribute portion for each institute.

The code registration as described above is performed by input at the manager terminal 400 itself or is performed by receiving approval information inputted through the manager terminal 40 with regard to codes requested to be registered by the operator terminal 300. The respective codes are registered so as to belong to at least one code group.

Meanwhile, the code management unit 108 has a function of receiving information of a code registration request transmitted from the operator terminal 300 in the case of code registration resulting from the registration request of the operator terminal 300; a function of maintaining the registration request information so as to be confirmed in the manager terminal 400; a function of receiving a command to inquire as to whether to the codes requested to be registered are pre-registered, which is inputted from the manager terminal 400, and providing an inquiry result based on the code table 310; and a function of receiving approval for new code registration information inputted from the manager terminal 400, registering corresponding codes in the code table 310, and maintaining the new codes in an available state in the user terminal 200. At this time, the manager terminal 400 becomes a terminal of a manager who has judged that the codes requested to be registered are non-registered codes.

Preferably, the code management unit 108 has a function of providing code registration information of the code table 310 to the operator terminal 300 so that inquiry can be performed as to whether or not the code requested to be registered is a pre-registered code, a function of receiving request information on reuse of an existing registered code or new code registration inputted through the operator terminal 300, and a function of registering the code requested for reuse as a code for each institute of a health institute to which the operator terminal 300 belongs when the request is a request for reuse of the existing registered code.

In addition, as illustrated in FIG. 2, the integrated management system for health information 100 according to the present embodiment further includes: a code table database 110 for keeping information on registered code groups and codes; a service information database 112 for keeping various kinds of request/search/handling/result/history matters and the like related with operational services; an analysis material database 114 for keeping result or statistical data created as a typical or atypical table; a user database 116 for keeping user information and authority related matters; an operator database 118 for keeping operator information and authority related matters; and a manager database 120 for keeping manager information and authority related matters.

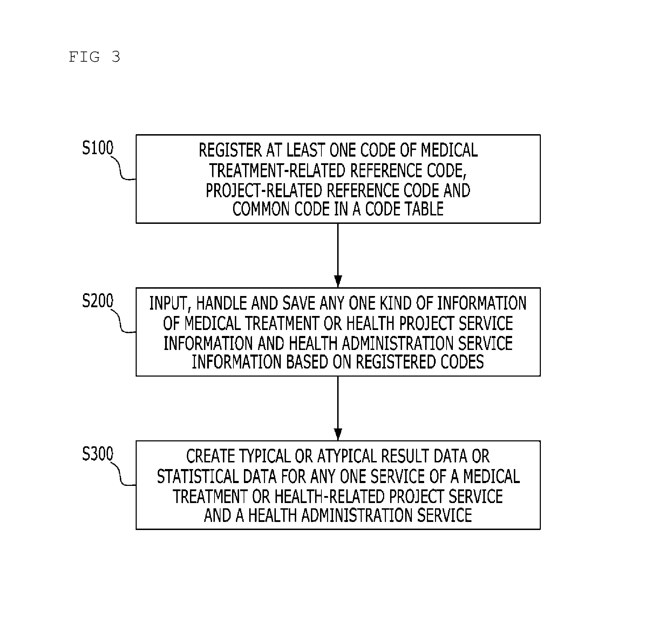

FIG. 3 is an entire flow chart showing an integrated management system for health information according to one embodiment of the present invention.

In order to integrally manage information on at least one service of medical treatment or health projects and health administration, at least one kind of medical treatment-related reference codes, reference codes for projects and common codes is registered in a code table (S100). Table 22 shows one example for the configuration of a basic code table with regard to the integrated management system for health information according to one embodiment of the present invention.

**[Table 22]**

| | | | |
|---|---|---|---|
| Division of Codes | | Name of Tables | Description of Tables |
| Common codes | | TCC_CMNCD_M | Common code master |
| | | TCC_PHC_M | Information on health institutes |
| Medical treatment-related reference codes | Fee information | TCC_FEEMM | Fee information |
| | Drug by injection | TSD_DRUGBASINFO_M | Basic information on drugs administered by injection |
| | Examination information | TSS-EXMBASINFO_M | Basic information on examination |
| | Terminology | TCC_KCD_M | Wound and disease master |
| | | TMR_VOC_M | Terminology master |
| Service codes | | THA_VCCINFMAP_I | Vaccine information for each institute |
| | | TCH_DZGRPDZSET_M | Setting of groups for classification of chronic diseases |

The registration of these codes is realized by input at the manager terminal itself or is realized by receiving approval inputted through the manager terminal with regard to codes for which registration is requested from the operator terminal. The respective codes are registered so as to belong to at least one code group.

The medical treatment related reference codes have a hierarchical structure composed of single codes defining an item corresponding to one remuneration processing unit, groups defined by a combination of the single codes, and a profile defined by a combination of at least two groups or single codes.

Also, the medical treatment-related reference codes are configured so as to use the profile at the time of inputting, processing and saving information on the health projects.

The single codes include a first single code defining an object item targeted for insurance benefits corresponding to an insurance code and a second single code defining an item that is not based on the insurance code and is added as a single code object through approval information inputted from the manager terminal.

In another aspect, the medical treatment-related reference codes are composed of an attribute portion for a whole region whose management authority is possessed by the manager terminal, and an attribute portion for each institute whose management authority is possessed by the operator terminal for each health institute.

Next, inputting, processing and saving one kind of service information of medical treatment or health-related project service information and health administration service information are performed through the user terminal based on the codes (S200). This process may be understood as a so-called processing process for operational services. At this time, the codes are codes for whole regions defined so as to be used by all user terminals of health institutes, or codes for each institute defined so as to be used by the user terminals of each health institute.

More preferably, in addition to processing of the operational services, result data or statistical data prepared as a typical or atypical table regarding one service of a medical treatment service, a health-related project service and a health administrative service is generated (S300).

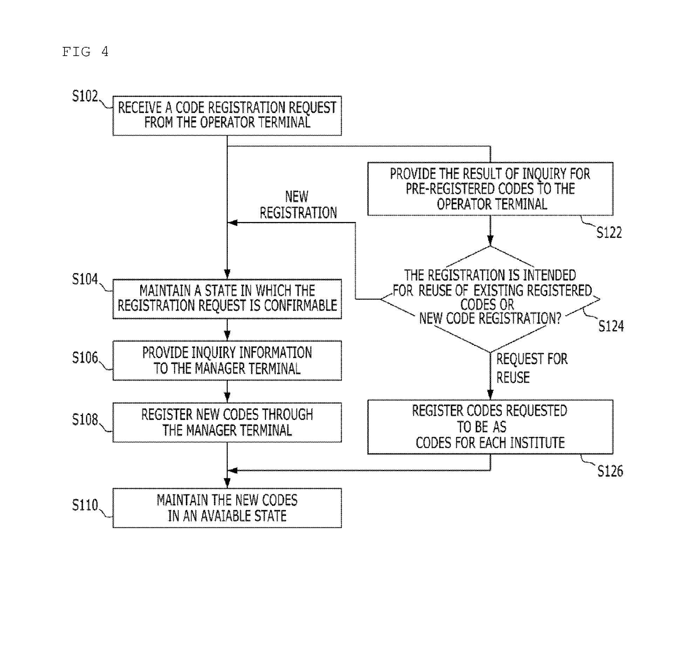

FIG. 4 is a flow chart showing a code registration process resulting from a request for code registration transmitted from the operator terminal with regard to the integrated management method for health information according to one embodiment of the present invention. In the code registration process of step S100 above, in particular, the code registration performed by a registration request of the operator terminal is performed as follows.

The integrated management system for health information receives a request for code registration transmitted from the operator terminal (S102).

The request for registration is maintained so as to be confirmed in the manager terminal (S104).

A command to inquire as to whether or not the codes requested to be registered are pre-registered is inputted and received from the manager terminal, and a result thereof is provided based on the code table (S106).

By receiving approval for the new code registration information from the manager terminal, the corresponding codes are registered in the code table (S108), and the new codes are maintained in an available state in the user terminal (S110). At this time, the manager terminal becomes a manager terminal which judges that the code requested to be registered is a non-registered code.

Table 23 shows an exemplary configuration of screen menus for the input of code registration of the manager terminal with regard to the integrated management system for health information according to one embodiment of the present invention.

**[Table 23]**

| Classification of Menus (Examples) | Inputted Menus (Examples) |
|---|---|
| Cost code-related menu | Cost (drug) code, name of costs (drugs) and insurance code |
| Insurance-related menu | Large classification/middle classification/small classification of insurance and the division of groups |
| Drug-related menu | Division of drugs, division of costs, and exception or non-exception of separation of dispensing and prescribing drugs |

For example, a manager who receives a request for code registration carries out the search for a code requested to be registered using a drug name through a menu input screen of the manager terminal showing [information search]->[search for special drugs]. When a drug related with the code is a drug for insurance benefits, the code of which has already been registered, the manager carries out a search for the drug from all registered drugs using an insurance code and registers the code so as to be used in a relevant health institute through a download function. Meanwhile, when the drug is a drug has no insurance benefits, the code of which is not registered, the manager accurately confirms a drug name and pharmaceutical company to be registered and register the code so as to be used in health institutes after generating the code as a new code.

Meanwhile, more preferably, after step S104 above, the code registration information of the code table is provided to the operator terminal so that inquiry as to whether the code requested to be registered is a pre-registered code can be performed (S122).

Request information on reuse of an existing registered codes or new code registration is inputted through the operator terminal (S124).

When the request is a request for reuse of the existing registered codes, the code requested to be registered is registered as a code for each institute of a health institute to which the operator terminal belongs (S126).

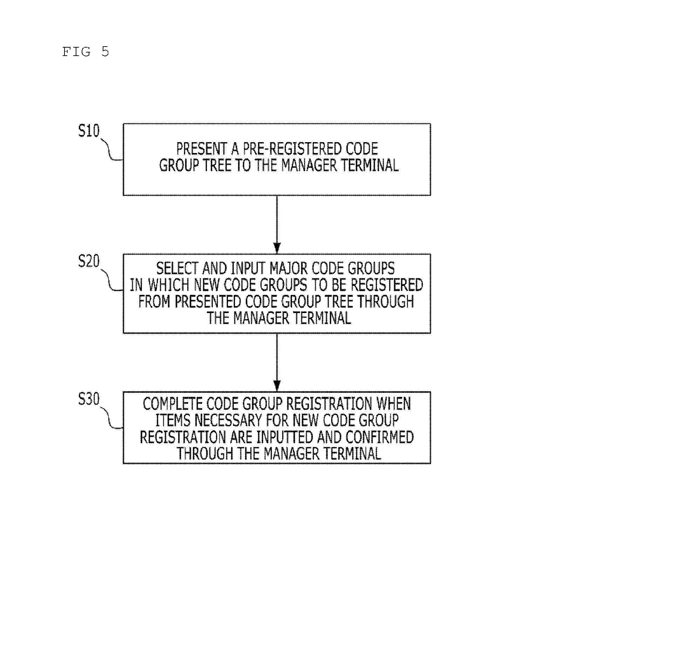

FIG. 5 is a flow chart showing the registration of code groups with regard to an integrated management method for health information according to one embodiment of the present invention. The registration of the code groups is performed before step S100 above. The registration process of the code groups is as follows.

A pre-registered code group tree is presented to the manager terminal (S10). The code group tree is hierarchized in code groups of at least two classes.

The major code groups in which new code groups will be registered are selected from the presented group code tree through the manager terminal (S20).

When items necessary for the registration of new code groups are inputted and confirmed through the manager terminal, code group registration is completed (S30).

Table 24 shows one example for the configuration of menus of a screen for code group registration with regard to the integrated management system for health information according to one embodiment of the present invention.

**[Table 24]**

| Classification of Codes (Examples) | Inputted Items (Examples) |
|---|---|
| Terminology code | ID(Identification), categories, type of groups, name of groups, classification of management, rules for code generation and the like |
| Common code | |
| Medical treatment code | |

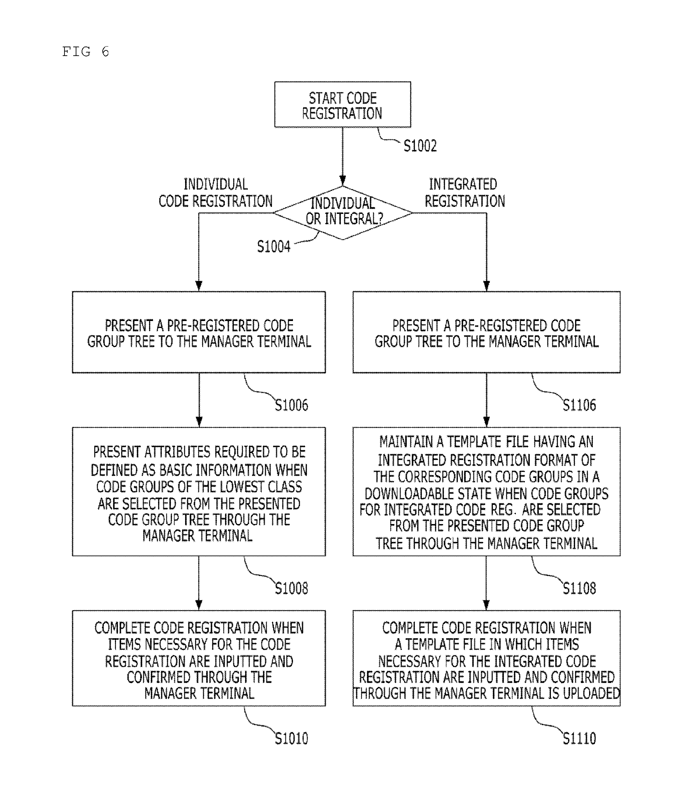

FIG. 6 is a flow chart showing individual or integrated code registration with regard to the integrated management method for health information according to one embodiment of the present invention.

The code registration may be selectively performed by two methods such as individual code registration or integrated code registration (S1002∼S1004).

First, a code registration process based on the individual code registration will be described.

A pre-registered code group tree is presented to the manager terminal (S1006). The code group tree is hierarchically organized in code groups of at least two classes.

When a code group of the lowest class is selected from the presented code group tree through the manager terminal, attributes needed to be defined for code registration in the corresponding code group are presented as basic information (S1008).

When items necessary for code registration are inputted and confirmed through the manager terminal, the code registration is completed (S1010).

Next, a code registration process based on the integrated code registration will be described.

A pre-registered code group tree is presented to the manager terminal (S1106). The code group tree is hierarchically organized in code groups of at least two classes.

When a code group required to be integrally registered is selected from the presented code group tree through the manager terminal, a template file having an integrated registration format of the corresponding code group is maintained in a downloadable state (S1108).

When the template file in which items necessary for integrally registering codes is inputted and uploaded from the manager terminal, the registration of the codes is completed (S1110).

The template file may be composed in, for example, a general spreadsheet file form and may include various kinds of information on the name of code groups, the name of codes, attributes and the like in a table form.

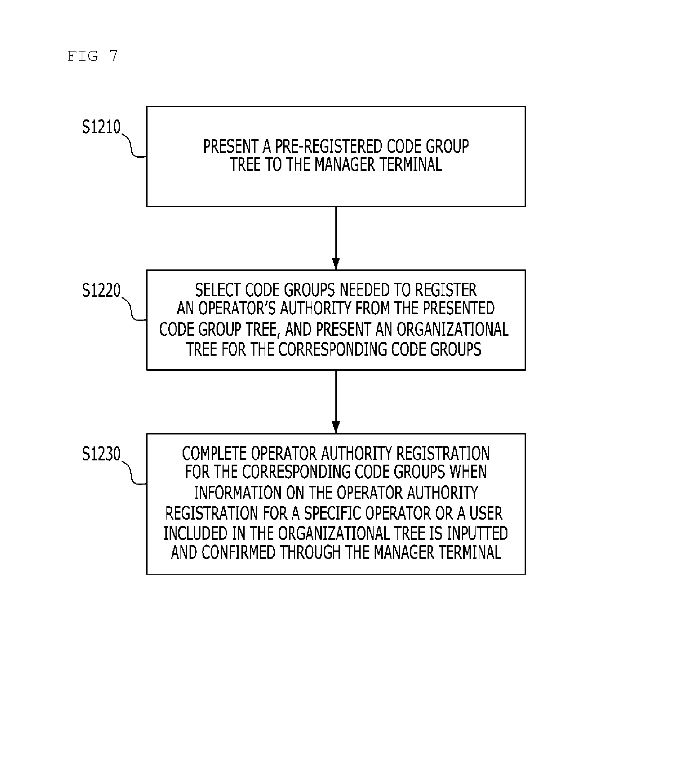

FIG. 7 is a flow chart showing the registration of an operator's authority with regard to the integrated management method for health information according to one embodiment of the present invention. The registration of the operator's authority to register, modify and delete codes according to each code group which can be requested using the manager terminal is performed, and the process thereof is as follows.

A pre-registered code group tree is presented to the manager terminal (S1210). The code group tree is formed in a hierarchical structure.

A code group needed to register an operator's authority is selected from the presented code group tree through the manager terminal, and an organizational tree for the corresponding code group is presented (S1220). The organizational tree is configured such that an organization including an operator or a user of the code group is hierarchically organized.

When information on operator authority registration for the specific operator or the user included in the organizational tree is inputted and confirmed through the manager terminal, the operator authority registration in the corresponding code group is completed (S1230). The information on the operator authority registration may be one kind of information of new operator registration information and existing operator deletion information.

Table 25 shows one example for the configuration of screen menus intended for managing and registering an operator's authority of the integrated management system for health information according to one embodiment of the present invention.

**[Table 25]**

| Classification of Codes (Examples) | Inputted Items (Examples) |
|---|---|
| Terminology code | User search, local selection, user selection, power level and the like |
| Common code | |
| Medical treatment code | |

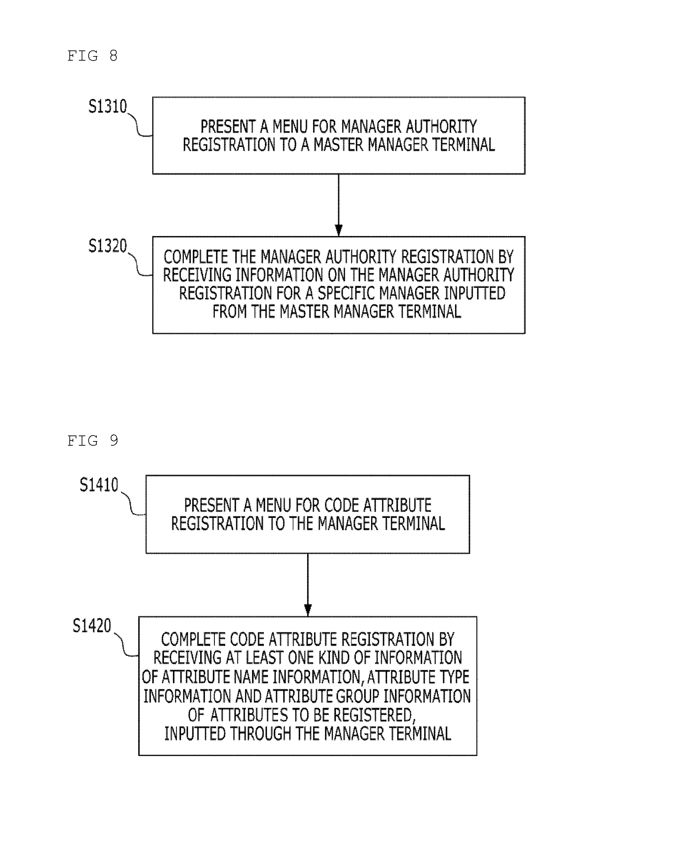

FIG. 8 is a flow chart showing the process of registering a manager's authority with regard to the integrated management method for health information according to one embodiment of the present invention. The registration of the manager's authority to register, modify and delete codes according to each code group may be performed, and the process thereof is as follows.

A menu for the registration of the manager's authority is presented to the master manager terminal (S1310).

The registration of the manager's authority for a specific manager is inputted from the master manager terminal, so that the registration of the manager's authority is completed (S1320). Information on the registration of the manager's authority may be one of information on the registration of a new manager and information on the deletion of an existing manager.

FIG. 9 is a flow chart showing the registration of code attributes with regard to the integrated management method according to one embodiment of the present invention. The registration of the code attributes is performed prior to step S100 above and the registration of the code attributes is carried out in the following process.

A menu for the registration of code attributes is presented to the manager terminal (S1410).

The code attribute registration is completed by receiving at least one kind of information of attribute name information, attribute type information and attribute group information to be registered, inputted from the manager terminal (S1420).

Table 26 exemplifies some types of the attributes.

**[Table 26]**

| Attribute Type | Type Name of Code Attribute | Example of Attribute Value |
|---|---|---|
| STRN | String | Notebook computers |
| NVAL | Attribute type having simple numeric values | |
| LSTN | Line break string | Characteristic of notebook computers - high performance |
| ENUM | Attribute type having input values | |

For example, based on attribute values for each attribute, attribute types in a determined number are supported. The attributes must have one type among the attribute types in the determined number, and such a defined type cannot be changed. As the attribute type is defined, setting of a unit and an input item is changed. An attribute having an attribute type of a numerical type must have a unit, and in the case of an attribute of an ENUM type, an input value must be additionally defined.

An attribute group is a group showing an attribute and is a classification standard for attributes intended to show item information by grouping items whose attributes have similar meanings at the time of presenting the item information.

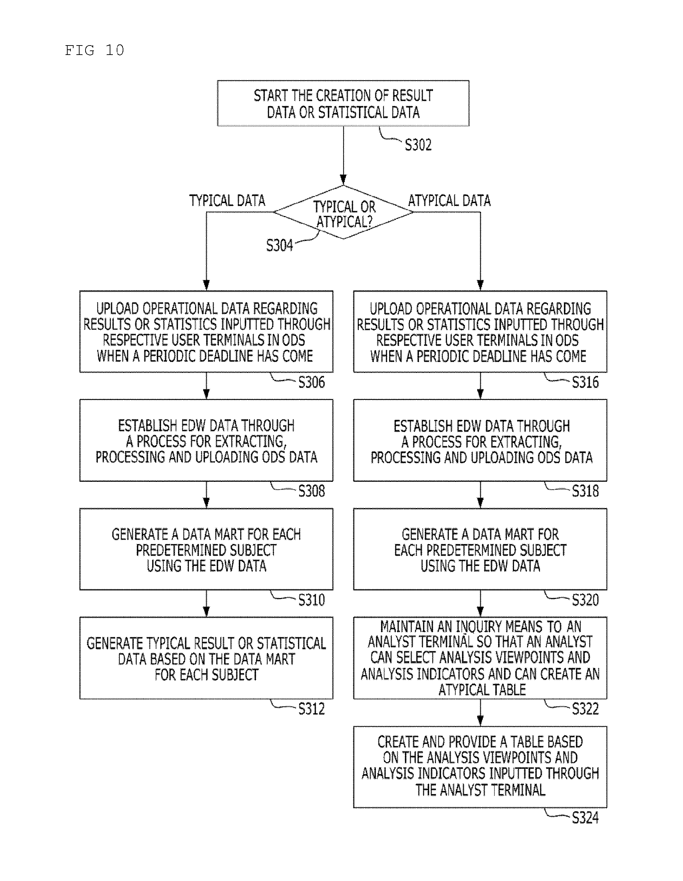

FIG. 10 is a flow chart showing the creation of a typical or atypical table with regard to the integrated management method according to one embodiment of the present invention.

In the integrated management method for health information of the present embodiment, as described above, in addition to the handling (various kinds of request/inquiry/result saving/history saving, etc. with regard to the respective detailed services) of operational services, such as medical treatment or health projects, health administration and the like, result data or statistical data for any one kind of service of medical treatment or health-related project services and health administration services prepared as a typical or atypical table is created (S302∼S304).

Here, 'the typical table' may refer to a table created so as to be used by saving a frequently used table and inputting a condition value and may have an analysis function in a form in which an inquiry item is partially changed or an analysis axis is partially changed using an additional function.

An 'atypical table' may refer to a table created by an analyst freely selecting analysis viewpoints and analysis indicators. When an atypical table is used, the analyst may create a report by combining the analysis viewpoints and analysis indicators (numerical value) in various respects, and the atypical table is advantageous in that detailed data inquiry and various analysis viewpoints can be provided. In order to create this atypical table, an atypical analysis template may be used, and a limited ad-hoc query in a form in which frequently used analysis viewpoints and analysis indicators are grouped in advance so as to be provided to analysts and necessary items are selected may be provided. The atypical analysis template may be understood as the concept of a semi-structured table in another aspect.

For the analysis as described above, the integrated management system for health information has an analysis data generating unit configured to generate result data or statistical data prepared as a typical or atypical table for services using operational service information, wherein the analysis data generating unit has an ODS processing unit, an EDW processing unit and a mart processing unit according to each detailed function.

First, a generation process for the typical table will be described.

When a periodic deadline has come, result or statistics-related operational data inputted through the respect user terminals are uploaded in an ODS (Operational Data Store) (S306). The ODS has the same layout as that of the operational data and stores the operational data as volatile data having temporary workability before uploading the operation data in an EDW.

An EDW (Enterprise Data Warehouse) data is established through a process for extracting, processing and uploading the ODS data (S308). The EDW includes operational data and historical data, manages refined columns and data with regard to code columns and has a state in which an integrated table for specific subjects (e.g., civil petitioners, fees, a receiving teller's window, and integrated master and history information) exists.

A data mart for each predetermined subject is generated using the EDW data (S310). The data mart is created by uploading data based on the EDW data and corresponds to a basic table for typical/atypical table services. In order to create the data mart through the EDW, a dimension may be used. With regard to the dimension, code information is managed as a table. General concepts of the ODS, the EDW, the data mart and the dimension and the like may be understood through many materials which are publicly known.

Based on the data mart for each subject, result or statistical data prepared as a typical table is automatically created (S312).

Table 27 shows exemplary items of a result data screen for the integrated management system for health information according to one embodiment of the present invention, namely, the examples of items which can be included in the created table.

**[Table 27]**

| Result (Example) | Configuration of Result Data Screen (Example) |
|---|---|
| Tuberculosis management project | Form the number of protective inoculation cases/the number of found patients, and the like for each local health center as tables |
| Current state of patients according to days of the week | Analyze the current state and the number of patients using the trend of graphs |
| Statistics for each treatment item | Form the current state of prescription for each drug as tables according to each day of the week/month to month/cumulative total |
| Statistics for medical checkups | Collect statistics for men/women patient types such as obesity/high blood pressure/anemia, etc. according to each region |

Next, the generative processes of atypical tables are explained.

When a periodic deadline has come, result-related or statistics-related operational data inputted from each user terminal is uploaded in an ODS (Operational Data Store) (S316).

EDW (Enterprise Data Warehouse) data is established through processes for extracting, processing and uploading the ODS data (S318).

A data mart for each pre-established subject is generated using the EDW data (S320).

An inquiry means is maintained by the analyst terminal so that an atypical table can be created by the analyst selecting the analysis viewpoints and the analysis indicators (S322).

Based on the analysis viewpoints and the analysis indicators inputted from the analyst terminal, the table is created and provided (S324).

Table 28 below exemplifies requirements for analysis of the analyst.

**[Table 28]**

| Institutes | Purpose of Utilization for Each Institute | Examples of Requirements |
|---|---|---|
| Health institute | Calculate results and statistics according to the conduction of business of health institutes | Free medical treatment results according to each month to month and each age - current state of medical treatment for each chronic disease (hypertension, diabetes, etc.) - materials submitted according to audit demands (audits of local governments or government offices) |
| Local government | Calculate the results of competent health institutes and statistics relating to local health care | The number of medical treatment cases according to each health institute and each doctor, and the monthly current state of medical treatment for a specific year according to each health institute |
| The Ministry of Health-Welfare | Calculation of various results and statistics based on health care policy establishment | The details of medical treatment according to each region of the country and the current state of visits of health institutes according to each age |

For example, this table may be generated in such a manner that in order of an operational database, ODS, a dimension and a mart, a table for protective inoculation results is generated based on fee information of medical treatment-related reference codes, or in order of an operational database, an ODS, dimension 1, dimension 2 and a mart, a table for the details of tests is created based on a profile of the medical treatment-related reference codes.

Table 29 exemplifies mart items for each subject with regard to the integrated management system for health information according to one embodiment of the present invention.

**[Table 29]**

| Division of Services (Examples) | Mart. (Examples) | Description |
|---|---|---|
| Health promotion | Current state of professional manpower arrangement | Arrangement state of exercise specialists/dietitians |
| Common education | Health education | The number of times of training, the number of Persons and educational activity results for each health project |
| Oral cavity | Mouth treatment | Current state analysis of oral treatment |
| Elderly Persons | Registration of dementia | Analysis of registered dementia patients |
| Chronic disease | Managed subjects for chronic diseases | Analysis of patients targeted for chronic disease management according to each disease |

Table 30 exemplifies the configuration of screen menus to be inputted for the creation of an atypical table with regard to the integrated management system according to one embodiment of the present invention.

**[Table 30]**

| Order | Menu contents inputted for the creation of an atypical table (Example) |
|---|---|
| 1 | Select a project targeted for the creation of the atypical table (e.g., health, oral cavity, an infectious disease, etc.) |
| 2 | Select an atypical list according to each detailed subject (in the case of an infectious disease, examination, the details of examination, a protective inoculation result, etc.), and select analysis conditions/analysis indicators |
| 3 | Select conditions for inquiry and inquiry items (e.g., the selection of a region to be inquired and the selection of an inquiry period) |
| 4 | Select analysis target indicators (e.g., the number of new families to be managed, the number of newly registered families, the number of withdrawn families, etc.) |
| 5 | Generate an atypical table according to inquiry conditions, analysis targets, and the like (e.g., generate the trend of changes by each period for respective analysis items in a table form) |

In the screen of order 1, the selection of inquiry items for each project is performed.

After this, the selection of an atypical list according to each detailed subject is performed in the screen of order 2. Here, the 'analysis conditions' mean items which can be added in terms of condition inquiry or screen analysis, and the 'analytical indicators' mean values (numerical values) for inquiring the selected conditions or analysis viewpoints.

Then, in the screen of order 3, conditions for inquiry and items to be inquired are selected, and in particular, the selection of basic conditions such as a period/organization and the like is carried out. In the screen of order 4, indicators (numerical values) to be analyzed and a viewpoint of analysis are selected.

After this, in the screen of order 5, a report in an atypical table form is created through report execution.

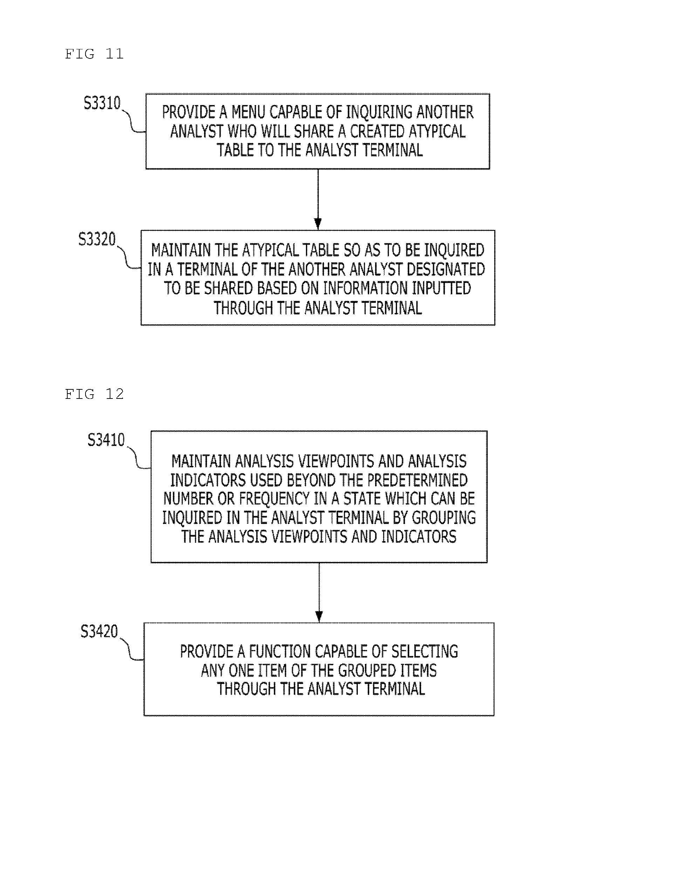

FIG. 11 is a flow chart showing an atypical table sharing process with regard to the integrated management method for health information according to one embodiment of the present invention. The atypical table may be shared among analysts through the following processes.

A menu for inquiring assistance of another analyst who may share the atypical table created as described above is provided to the analyst terminal (S3310).

Based on information inputted through the analyst terminal, the atypical table is maintained so as to be inquired about in another analyst terminal designated to share it (S3320).

FIG. 12 is a flow chart showing a grouping process for analysis viewpoints and analysis indicators with regard to the integrated management method for health information according to one embodiment of the present invention. The analysis viewpoints and analysis indicators used for creation of the atypical table may be grouped, saved and provided repeatedly through the following processes.

The analysis viewpoints and analysis indicators used beyond the predetermined number of times or a predetermined frequency are grouped so as to be maintained in an inquirable state in the analyst terminal (S3410).

The analyst terminal is provided with a function capable of selecting any one item among the grouped items (S3420).

The embodiments of the present invention include a computer readable recording medium including a program command to carry out motions implemented by various computers. The computer readable recording medium may include any or a combination of a program command, a data file, a data structure and the like. The medium is specially designed and configured for the present invention, but may be mediums that are publicly known and commonly used to those having ordinary skill in the art in the field of computer software. Examples of the computer readable recording medium include hard disks, floppy disks, magnetic mediums such as magnetic tapes, CD-ROMs, optical mediums such as DVDs, magnetic-optical mediums such as floptical disks, and hardware devices specially configured so as to save and perform program commands such as ROM, RAM, flash memory and the like. The medium may be a transmission medium of an optical or metallic wire, a waveguide and the like including carrier waves transmitting signals which designate the program commands, data structure and the like. Examples of the program command include high-level language codes which can be executed by computers using interpreters as well as machine language codes made by compilers.

Although the preferred embodiments of the present invention have been disclosed with reference to the accompanying drawings for illustrative purposes, those skilled in the art will appreciate that various modifications, additions and substitutions are possible, without departing from the scope and spirit of the invention. Accordingly, the scope and spirit of the invention should be interpreted based on the accompanying claims described in such a manner that these various modifications can be included.

## Claims

1. An integrated management method for health information, which is a method of integrally managing information on at least one service of a service for medical treatment or health-related projects and a service for health administration inputted or requested through a user terminal; and which is performed through an integrated management system for health information connected to at least a user terminal, an operator terminal and a manager terminal via a network, the method comprising:
step (a) of registering at least one kind of medical treatment-related reference codes, project-related reference codes, and common codes in a code table through execution of a code management unit, wherein the registering of the codes is performed through input at the manager terminal itself, or is performed by receiving approval information inputted through the manager terminal with regard to the codes requested to be registered by the operator terminal, and the respective codes are registered so as to belong to at least one code group; and
step (b) of inputting, processing and saving information on at least one service of a service for medical treatment or health-related projects and a service for health administration from the user terminal based on the codes according to execution of a service information processing unit, wherein the codes are codes for entire region defined so as to be used by all user terminals of health institutes or any one of codes for each institute defined so as to be used by the user terminals of each health institute.

2. The method of claim 1, wherein the medical treatment-related reference codes have a hierarchical structure composed of single codes defining an item corresponding to one remuneration processing unit, groups defined by a combination of the single codes, and a profile defined by a combination of at least two groups or single codes and is configured so as to use the profile at the time of inputting, processing and saving information on the health projects.

3. The method of claim 2, wherein the single codes comprise a first single code defining a subject item for insurance benefits corresponding to an insurance code and a second single code defining an item that is not based on the insurance code and is added as a subject for the single code by going through approval and input of the manager terminal.

4. The method of claim 1, wherein the registering of the codes resulting from a request for registration in said step (a) comprises: step (a1) of receiving information on the request for code registration transmitted from the operator terminal; step (a2) of maintaining the information on the request for registration so as to be confirmed through the manager terminal; step (a3) of receiving a command to inquire as to whether or not the codes requested to be registered are pre-registered, inputted from the manager terminal and then providing an inquiry result based on a code table; and step (a4) of receiving approval for information on the registration of new codes inputted from the manager terminal, registering corresponding codes in the code table, and maintaining the new codes in an available state in the user terminal, wherein the manager terminal is a terminal of a manager who judges that the codes requested to be registered are non-registered codes.

5. The method of claim 4, wherein after said step (a1), the registering of the codes comprises: step (aa1) of providing the code registration information of the code table to the operator terminal so that inquiry as to whether the code requested to be registered are pre-registered can be performed; step (aa2) of receiving request information for reuse of existing registered codes or new code registration inputted from the operator terminal; and step (aa3) of registering the codes requested for reuse as codes for each institution of a health institute to which the operator terminal belongs when the request is a request for reuse of the existing registered codes.

6. The method of claim 1, wherein the medical treatment related reference codes comprise at least one of an attribute portion for an entire region for which management authority is possessed by the manager terminal and an attribute portion for each institute for which management authority is possessed by the operator terminal of each individual health institute.

7. The method of claim 1, wherein before said step (a), registering of code groups is performed by execution of a code management unit, wherein the registering of the code groups comprises: step (pa1) of presenting a pre-registered code group tree to the manager terminal, the code group tree being hierarchically organized in code groups of at least two classes; step (pa2) of selecting major code groups for registering new code groups from the presented code group tree through the manager terminal; and step (pa3) of completing code group registration when items necessary for the registration of the new code group is inputted and confirmed through the manager terminal.

8. The method of claim 1, wherein said step (a) is performed by individual code registration, the individual code registration comprising: step (ai1) of presenting the pre-registered code group tree to the manager terminal, the code group tree being hierarchically organized in code groups of at least two classes; step (ai2) of presenting, as basic information, attributes required to be defined for code registration in corresponding code groups when a predetermined code group of the lowest class is selected from the presented code group tree through the manager terminal; and step (ai3) of completing the code registration when items necessary for the code registration are inputted and confirmed through the manager terminal.

9. The method of claim 1, wherein said step (a) is performed by integrated registration, the integrated registration comprising: step (at1) of presenting a pre-registered code group tree to the manager terminal, the code group tree being hierarchically organized in code groups of at least two classes; step (at2) of keeping a template file having an integrated registration format of corresponding code groups in a downloadable state when the code groups targeted for the integrated registration are selected from the presented code group tree through the manager terminal; and step (at3) of completing the code registration when the template file in which items necessary for the integrated registration are inputted through the manager terminal is uploaded.

10. The method of claim 1, which is configured so that the registration of an operator's authority to request code registration, modification and deletion for each code group to the manager terminal can be performed according to execution of the code management unit, the operator authority registration comprising: step (pb1) of presenting a pre-registered code group tree to the manager terminal, the code group tree being hierarchically organized in code groups of at least two classes; step (pb2) of selecting code groups requiring the registration of the operator's authority from the presented code group tree through the manager terminal and presenting an organizational tree for the corresponding code groups, the organizational tree being configured by hierarchically organizing an organization to which the operator or a user of the code groups pertains; and step (pb3) of completing the registration of the operator's authority for the corresponding code group when operator authority registration information for the specific operator or user included in the organizational tree is inputted and confirmed through the manager terminal, the operator authority registration information being at least one kind of information of new operator registration information or deletion of existing operator information.

11. The method of claim 1, which is configured so that the registration of a manager's authority to perform code registration, modification and deletion for each code group can be performed according to execution of the code management unit, the manager authority registration comprising: step (pc1) of presenting a menu for manager authority registration to a master manager terminal; and step (pc2) of receiving information on the manager authority registration for the specific manager inputted through the master manager terminal and completing the manager authority registration, the information on the manager authority registration being at least one kind of information of new manager registration information and information of existing manager deletion.

12. The method of claim 1, wherein before said step (a), code attribute registration is performed by execution of the code management unit, the code attribute registration comprising: step (pd1) of presenting a menu for the code attribute registration to the manager terminal; and step (pd2) of receiving at least one kind of information of attribute name information, attribute type information and attribute group information inputted from the manager terminal and completing the code attribute registration.

13. The method of claim 1, further comprising step (c) of generating result data or statistical data prepared as a typical or atypical table for one service of a medical treatment or health project service or a health administration service according to execution of an analysis data generating unit after said step (b).

14. The method of claim 13, wherein said (c) step comprises: step (ca1) of uploading result-related or statistics-related operational data inputted from each user terminal in an ODS (Operational Data Store) when reaching a periodic deadline; step (ca2) of establishing EDW (Enterprise Data Warehouse) data by going through processes for extracting, processing and uploading the ODS data; step (ca3) of generating a data mart for each pre-established subject using the EDW data; and step (ca4) of automatically generating result or statistical data prepared as a typical table based on the data mart for each subject.

15. The method of claim 13, where said (C) step further comprises: step (cb1) of uploading result-related or statistics-related operational data inputted from each user terminal in an ODS (Operational Data Store) when reaching a periodic deadline; step (cb2) of establishing EDW (Enterprise Data Warehouse) data by going through processes for extracting, processing and uploading the ODS data; step (cb3) of generating a data mart for each pre-established subject based on the EDW data; step (cb4) of maintaining an inquiry means to an analyst terminal so that an atypical table can be created as analyst selects an analysis point of view and an analysis index; and step (cb5) of creating the table based on the analysis point of view and the analysis index inputted from the analyst terminal and providing the created table.

16. The method of claim 15, further comprising: step (cb6) of providing a menu capable of checking another analyst for sharing the created atypical table to the analyst terminal; and step (cb7) of maintaining the atypical table so as to be checked in another analyst terminal designated to be shared based on information inputted through the analyst terminal.

17. The method of claim 15, further comprising: step (cb8) of grouping the analysis viewpoint and analysis index used beyond the predetermined number of times or frequency in use and maintaining the grouped analysis viewpoints and analysis index in a checkable state in the analyst terminal; and step (cb9) of providing a function capable of selecting any one item of the grouped items through the analyst terminal.

18. A computer readable recording medium, which is intended for implementing an integrated management method for health information according to any one of claims 1 to 17 using a computer, and in which a computer program is recorded.

19. An integrated management system for health information, which is connected to at least a user terminal, an operator terminal and a manager terminal via a network, and which is a system configured to integrally manage at least one kind of service information of medical treatment or health-related project service information and health administration service information inputted or requested through the user terminal,
the system, comprising:
a service information processing unit configured to handle one kind of service information of the medical treatment or health-related project service information and the health administration service information inputted or requested through the user terminal;
an analysis data generating unit configured to generate result data or statistical data prepared as a typical or atypical table on services using the service information;
a portal unit linked with a general user terminal intended for using a service processing function of the service information processing unit or using data generated from the analysis data generating unit and configured to handle inputted information or provide requested information; and
a code management unit configured to handle the registration, modification and deletion of codes for securing the compatibility of information processed by the service information processing unit, the analysis data generating unit and the portal unit.

20. The system of claim 19, wherein the code management unit at least carries out a function of registering at least one kind of medical treatment-related reference codes, project-related reference codes and common codes in a code table, wherein the registering of the codes is performed by input at the manager terminal itself or is performed by receiving approval information inputted through the manager terminal with regard to the codes requested to be registered by the operator terminal, and the respective codes are registered so as to belong to at least one code group.

21. The system of claim 20, wherein the medical treatment-related reference codes have a hierarchical structure composed of single codes defining items corresponding to one remuneration processing unit, groups defined by a combination of at least two single codes, and a profile defined by a combination of at least two groups or at least two single codes, and are configured so that the profile can be used at least at the time of inputting, processing and saving information on health-related project services.

22. The system of claim 21, wherein the single codes comprise: a first single code defining an object item targeted for insurance benefits corresponding to an insurance code; and a second single code defining an item not based on the insurance code and added as an object of the single code through approval information inputted from the manager terminal.

23. The system of claim 20, wherein the code management unit has a function of receiving information on a code registration request transmitted from the operator terminal in the case of code registration resulting from the registration request of the operator terminal; a function of maintaining the registration request information so as to be confirmed in the manager terminal; a function of receiving a command to inquire as to whether to the codes requested to be registered are pre-registered, which is inputted from the manager terminal, and providing an inquiry result based on the code table; and a function of receiving approval for new code registration information inputted from the manager terminal, registering corresponding codes in the code table, and maintaining the new codes in an available state in the user terminal, wherein the manager terminal becomes a terminal of a manager who has judged that the codes requested to be registered are non-registered codes.

24. The system of claim 23, wherein the code management unit has: a function of providing code registration information of the code table to the operator terminal so that inquiry as to whether or not the code requested to be registered is a pre-registered code can be performed; a function of receiving request information on reuse of an existing registered code or new code registration inputted through the operator terminal; and a function of registering the code requested for reuse as a code for each institute of a health institute to which the operator terminal belongs when the request is a request for reuse of the existing registered code.

25. The system of claim 20, wherein the medical treatment-related reference codes comprise at least one of an attribute portion for entire regions whose management authority is possessed by the manager terminal, and an attribute portion for each institute whose management authority is possessed by the operator terminals according to each health institute.
